# EUROPÄISCHE PATENTANMELDUNG

(11) **EP 3 418 284 A1**
(43) Veröffentlichungstag der Anmeldung: **26.12.2018**
(21) Anmeldenummer: 18182552.2
(22) Anmeldetag: 18.07.2014
(51) Int. Cl.: C07F 9/50, C07F 15/00, C07C 231/12, C07C 233/15

(54) **PHOSPHINLIGANDEN FÜR VERBESSERTES VERFAHREN ZUR HERSTELLUNG VON CHLORIERTEN BIPHENYLANILIDEN UND BIPHENYLANILINEN**

(30) Priorität: 23.07.2013 EP 13177583
(62) Teilanmeldung aus: 14741287.8
(71) Anmelder: Bayer CropScience Aktiengesellschaft, 40789 Monheim am Rhein (DE)
(72) Erfinder: DOCKNER, Michael, 50674 Köln (DE); RODEFELD, Lars, 51375 Leverkusen (DE); HEINRICH, Jens Dieter, 51381 Leverkusen (DE)
(74) Vertreter: BIP Patents

(57) **Zusammenfassung**

Die vorliegende Erfindung betrifft Verbindungen der Formel (V) 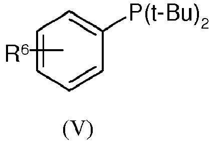 oder Salze hiervon.

## Beschreibung

Die Erfindung betrifft Phosphinliganden und deren Salze, die für ein verbessertes Verfahren zur Herstellung von halogenierten Biphenylaniliden und Biphenylanilinen durch Suzuki-Kupplung von Brom- oder Jodaniliden bzw. Brom- oder Jodanilinen mit chlorierten Organoborverbindungen eingesetzt werden können. Biphenylanilide und Biphenylaniline dienen als Vorstufen zur Herstellung von Pflanzenschutzmitteln mit fungizider Wirkung.

Durch Palladium katalysierte Kreuzkupplung halogenierter Aromaten der Formel (II) mit Organoborverbindungen der Formel (III) gewinnt man nach WO 2006/092429, WO 2007/138089 und WO 2009/135598 sowohl halogenierte Biphenylaniline als auch halogenierte Biphenylanilide der allgemeinen Formel (I) nach dem folgenden Schema 1. 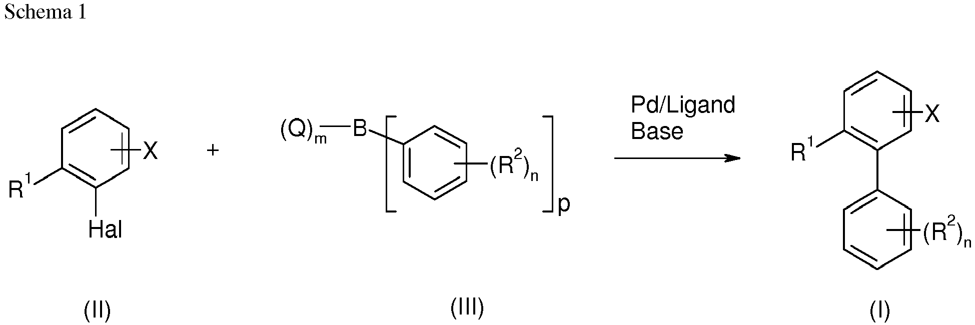 wobei die Substituenten wie folgt definiert sind:
- X: ist Wasserstoff, Fluor oder Chlor;
- Hal: ist Halogen
- R¹: ist ausgewählt aus einer geschützten Aminogruppe, NO₂, NH₂ und NHR³;
- R²: ist ausgewählt aus Cyano, Nitro, Halogen, C₁-C₆-Alkyl, C₁-C₆-Alkenyl, C₁-C₆-Alkynyl, C₁-C₆-Alkoxy, C₁-C₆-Haloalkyl, (C₁-C₆-Alkyl)Carbonyl und Phenyl;
- R³: ist ausgewählt aus Wasserstoff, -CH₂-(C=O)CH₃, C₁-C₈-Alkyl, C₁-C₈-Alkenyl, C₁-C₈-Alkynyl und C₆-C₁₈-Aryl
- n: ist ausgewählt aus 1, 2 und 3,
- p: ist ausgewählt aus 1, 2, 3 und 4 und
- Q: ist ausgewählt aus Hydroxyl, F, Cl, Br, I, C₁₋₄-Alkyl-, C₆₋₁₀-Aryl-, C₁₋₄-Alkoxy- und C₆₋₁₀-Aryloxy, C₁₋₄-Alkoxy Resten, die zusammen mit dem Boratom, an welches sie gebunden sind, einen 5- oder 6-gliedrigen Ring bilden, der mit C₁₋₄-Alkyl-Resten substituiert sein kann
und die Verbindungen der Formel (III) unterschiedliche Organoborverbindungen umfassen.

Als Liganden für die im obigen Schema dargestellte Suzuki-Reaktion kommen unsubstituierte und substituierte Trialkyl- oder Triarylphosphine zur Anwendung. Darüber hinaus eignen sich nach WO 2001/042197 auch Dialkylarylphosphine als Liganden. Die Auswahl möglicher Phosphinliganden wird von WO 2004/052939, S. 34f um Phosphine erweitert, bei denen einer der Reste am Phosphor aus einem biphenylischen Grundgerüst besteht.

Stehen in der allgemeinen Formel (II) die Substituenten
Hal für Brom oder Iod, und
X für Wasserstoff, Fluor oder Chlor, 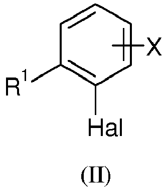 und der Substituent R² in der allgemeinen Formel (III) für Chlor, 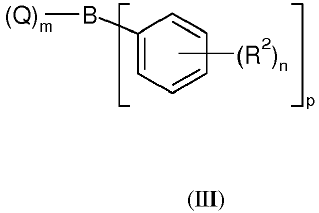 so reagiert unter den Bedingungen der Suzuki-Reaktion gebildetes, chloriertes Biphenylanilid bzw. Biphenylanilin mit Borverbindungen der allgemeinen Formel (III) weiter zu Triarylen der allgemeinen Formel (IV) und seinen höheren Homologen (Schema 2). Daraus resultiert ein Ausbeuteverlust an dem gewünschten Biphenylanilid bzw. Biphenylanilin der allgemeinen Formel (I).

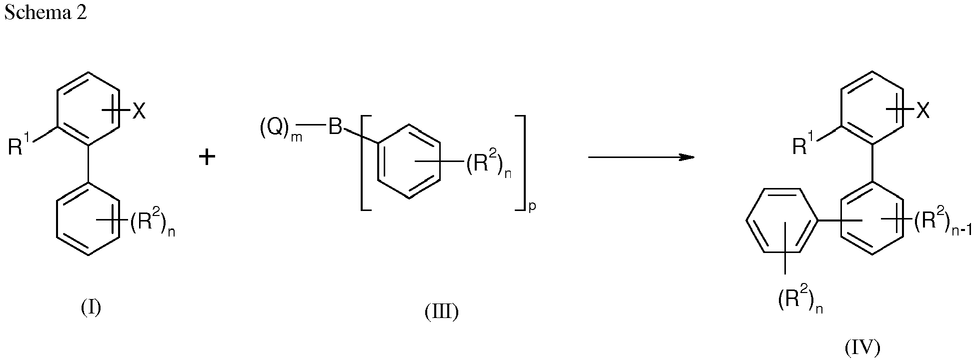

In welchem Ausmaß diese Folgereaktion stattfindet, bestimmt im Wesentlichen der in der Reaktion eingesetzte Phosphinligand.

WO 2004/052939 beschreibt die Verwendung von biphenylischen Phosphinen und Di(tert.-butyl)phenylphosphin, die die Suzuki-Reaktion von den im Vergleich zu Arylbromiden weniger reaktiven Arylchloriden mit Phenylboronsäuren selbst unter sehr milden Bedingungen katalysieren. Es ist in einem solchen Fall davon auszugehen, dass Triaryle in einem merklichen Ausmaß gebildet werden.

Durch die Einführung einer Schutzgruppe am Stickstoffatom gelingt es nach WO 2009/135598, den Anteil an Triarylen zu vermindern. In der Suzuki-Reaktion können sowohl Trialkyl- als auch Triarylphosphine eingesetzt werden.

Aufgabe der vorliegenden Erfindung war es, einen Liganden zu finden, der Triaryle nicht oder in einem sehr geringen Maß entstehen lässt und so einen wirtschaftlicheren Zugang zu chlorierten Biphenylaniliden bzw. Biphenylanilinen ermöglicht.

Die Aufgabe wird überraschend gelöst durch Phosphinliganden der Formel (V), die wie folgt definiert sind 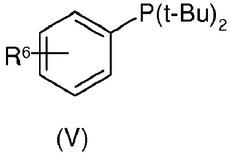 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂
oder ein Salz hiervon.

Diese können beispielsweise eingesetzt werden in einem verbesserten Verfahren zur Herstellung von chlorierten Biphenylaniliden und Biphenylanilinen der Formel (I) 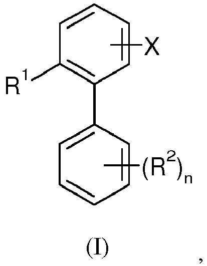 wobei die Substituenten wie folgt definiert sind:
- X: ist ausgewählt aus Wasserstoff, Fluor und Chlor;
- R¹: ist ausgewählt aus einer geschützten Aminogruppe, NO₂, NH₂ und NHR³;
- R²: ist Chlor;
- R³: ist ausgewählt aus Wasserstoff, -CH₂-(C=O)CH₃, C₁-C₈-Alkyl, C₁-C₈-Alkenyl, C₁-C₈-Alkynyl und C₆-C₁₈-Aryl.
- n: ist ausgewählt aus 1, 2 und 3,
durch Umsetzung von Halogenaromaten der allgemeinen Formel (II) 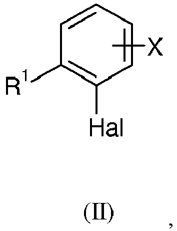 worin
- Hal: ausgewählt ist aus Brom und Jod; und X und R¹ wie oben definiert sind,
in Gegenwart einer Base und eines Palladium Katalysators ausgewählt aus der Gruppe bestehend aus
a) einem Komplex bestehend aus Palladium in der Oxidationsstufe 0 und einem Phosphinliganden der Formel (V) oder einem Salz hiervon,
b) einem Palladiumsalz in Gegenwart eines Phosphinliganden der Formel (V) oder einem Salz hiervon und
c) metallischem, gegebenenfalls auf einen Träger aufgebrachtem Palladium, in Gegenwart eines Phosphinliganden der Formel (V) oder einem Salz hiervon,
wobei der Phosphinligand der Formel (V) wie zuvor beschrieben definiert ist 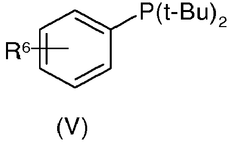 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂
oder ein Salz hiervon,
in einem Lösungsmittel, mit einer Organoborverbindung der Formel (III) 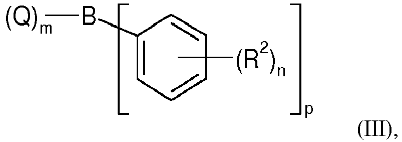 ausgewählt aus der Gruppe bestehend aus:
(i) Boronsäuren der Formel (III) in welchen
   - m: 2 ist,
   - p: 1 ist,
   - Q¹ und Q²: Hydroxylgruppen sind,
   - R² und n: wie oben definiert sind,
   oder die aus den Boronsäuren der Formel (III) gebildeten Anhydride, Dimere oder Trimere;
(ii) Boronsäurederivate der Formel (III), in welchen
   - m: 2 ist,
   - p: 1 ist,
   Q¹ und Q² jeweils unabhängig voneinander ausgewählt sind aus F, Cl, Br, I, C₁₋₄-Alkyl-, C₆₋₁₀-Aryl-, C₁₋₄-Alkoxy- und C₆₋₁₀-Aryloxy sind,
   R² und n wie oben definiert sind;
(iii) Borinsäuren der Formel (III), in welchen
   - m: 1 ist,
   - p: 2 ist,
   - Q: ausgewählt ist aus OH, F, Cl, Br, I, C₁₋₄-Alkyl-, C₆₋₁₀-Aryl-, C₁₋₄-Alkoxy- und C₆₋₁₀-Aryloxy-Resten,
   - R² und n: wie oben definiert sind;
(iv) cyclische Boronsäureester der Formel (III), in welchen
   - m: 2 ist,
   - p: 1 ist,
   - Q¹ und Q²: jeweils unabhängig voneinander ausgewählt sind aus C₁₋₄-alkoxy Resten, die zusammen mit dem Boratom, an welches sie gebunden sind, einen 5- oder 6-gliedrigen Ring bilden, der mit C₁₋₄-Alkyl-Resten substituiert sein kann,
   - R² und n: wie oben definiert sind;
(v) Boronate der Formel (III), in welchen
   - m: 3 ist,
   - p: 1 ist,
   - R² und n: wie oben definiert sind,
   - Q¹ bis Q³: jeweils unabhängig voneinander ausgewählt sind aus OH, F, Cl, Br, I, C₁₋₄-Alkyl-, C₆₋₁₀-Aryl-, C₁₋₄-Alkoxy- und C₆₋₁₀-Aryloxy-Resten,
   und worin die negative Ladung des Boronat Anions durch ein Kation kompensiert ist;
(vi) Triarylborane der Formel (III), in welchen
   - m: 0 ist,
   - p: 3 ist,
   - R² und n: wie oben definiert sind;
(vii) Tetraarylborate der Formel (III), in welchen
   - m: 0 ist,
   - p: 4 ist,
   - R² und n: wie oben definiert sind,
   und in welchen die negative Ladung des Boronat Anions durch ein Kation kompensiert ist.

Eine Variante des oben beschriebenen Verfahrens zur Herstellung substituierter Biphenylanilide der Formel (I) lautet 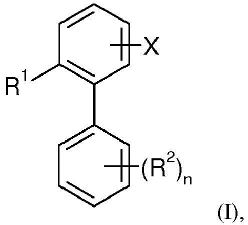 worin
- X: ausgewählt ist aus Wasserstoff, Fluor und Chlor;
- R¹: ausgewählt ist aus -NH(CO)R³, -N=CR⁴R⁵, NO₂, NH_{2,} und NHR³;
- R²: Chlor ist;
- R³, R⁴, R⁵: unabhängig voneinander ausgewählt sind aus Wasserstoff, -CH₂-(C=O)CH₃, C₁-C₈-Alkyl, C₁-C₈-Alkenyl, C₁-C₈-Alkynyl und C₆-C₁₈-Aryl; oder
- R⁴, R⁵: zusammen mit dem Kohlenstoffatom, an das sie gebunden sind einen 5- oder 6-gliedrigen Ring bilden können, der 1, 2 oder 3 Heteroatome ausgewählt aus N, O, und S enthält;
- n: ausgewählt ist aus 1, 2 und 3.

Gemäß einer Ausführungsform der vorliegenden Erfindung ist der Phosphinligand der Formel (V) eine Verbindung der Formel (V-i) 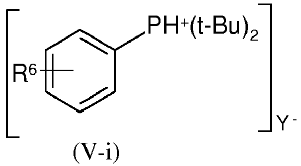 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂ und
- Y: ausgewählt ist aus der Gruppe bestehend aus BF₄⁻, Perchlorat und Hydrogensulfat.

In einer bevorzugten Ausführungsform ist die Verbindung der Formel (V-i) eine Verbindung der Formel [(t-Bu)₂PhPH]BF₄.

Überraschenderweise wurde gefunden, dass der Einsatz von Phosphinliganden der allgemeinen Formel (V) 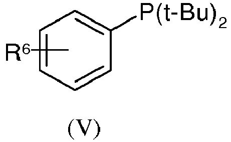 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂
oder ein Salz hiervon, in der Suzuki-Reaktion nach Schema 1 mit hoher Selektivität und geringem Ausmaß an Triarylbildung chlorierte Biphenylanilide bzw. Biphenylaniline liefert.

### Organobor Verbindungen

Die Organoborverbindungen, die bei dem oben genannten Verfahren verwendet werden können:
(i) Boronsäuren der Formel (III) in welchen
   - m: 2 ist,
   - p: 1 ist,
   - Q¹ und Q²: Hydroxylgruppen sind,
   - R² und n: wie oben definiert sind,
   oder die aus den Boronsäuren der Formel (III) gebildeten Anhydride, Dimere oder Trimere,
   sind durch Umwandlung von Arylmagnesiumhalogeniden mit Trialkylboraten, vorzugsweise in THF als Lösungsmittel, erhältlich. Zur Unterdrückung der Bildung von Arylborinsäuren ist es notwendig, keinen Überschuss der beiden Reagentien zu verwenden und die Umsetzung bei niedrigen Temperaturen von -60°C durchzuführen, wie in R.M. Washburn et al. Organic Syntheses Collective Band 4, 68, oder in Boronic Acids, Herausgeber Dennis G. Hall, Wiley-VCH 2005, S.28ff, und darin angegebenen Literaturstellen beschrieben.
   Als Beispiele für Boronsäuren, die gemäß der vorliegenden Erfindung verwendet werden können, seien die folgenden Verbindungen genannt:
   4-Chlorphenylboronsäure, 3-Chlorphenylboronsäure, 2-Chlorphenylboronsäure, 3,4-Dichlorphenylboronsäure und 2,3-Dichlorphenylboronsäure, insbesondere 3,4-Dichlorphenylboronsäure.
(ii) Boronsäurederivate der Formel (III), in welchen
   - m: 2 ist,
   - p: 1 ist,
   Q¹ und Q² jeweils unabhängig voneinander ausgewählt sind aus F, Cl, Br, I, C₁₋₄-Alkyl-, C₆₋₁₀-Aryl-, C₁₋₄-Alkoxy- und C₆₋₁₀-Aryloxy sind,
   R² und n wie oben definiert sind;
(iii) Borinsäuren der Formel (III), in welchen
   - m: 1 ist,
   - p: 2 ist,
   - Q: ausgewählt ist aus OH, F, Cl, Br, I, C₁₋₄-Alkyl-, C₆₋₁₀-Aryl-, C₁₋₄-Alkoxy- und C₆₋₁₀-Aryloxy-Resten,
   - R² und n: wie oben definiert sind,
   werden durch Umsetzung von gegebenenfalls substituiertem Phenylmagnesiumchlorid V mit Trialkylborat, vorzugsweise Trimethylborat, in Tetrahydrofuran als Lösungsmittel gemäß WO 2007/138089 erhalten, wie in Schema 3 beschrieben. 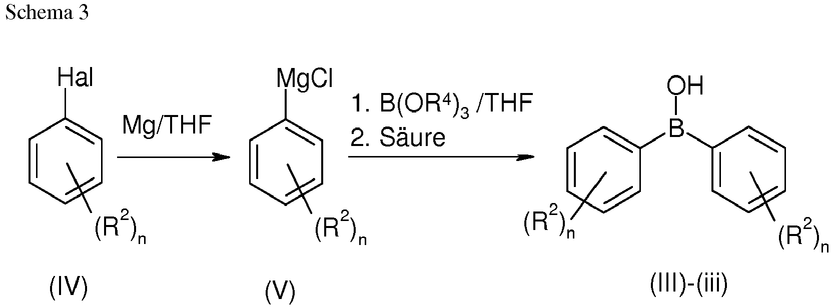
   - R⁴: steht für C₁-C₄-Alkyl, vorzugsweise Methyl.
   - Hal: steht für Cl, Br, I.
   Vorzugsweise geht man von Diphenylborinsäuren der Formel (iii) aus, worin m für 1 steht, p für 2 steht, Q für OH steht und R² und n die oben angegebenen Bedeutungen haben.
   Weitere Ausgangsstoffe sind Diphenylborinsäuren (iii), worin n für 1 oder 2, insbesondere 2, steht. Besonders bevorzugt sind Diphenylborinsäuren (iii), die in der 3- und 4-Position oder nur in der 4-Position substituiert sind.
   Borinsäuren, die gemäß der vorliegenden Erfindung verwendet werden können, sind ausgewählt aus der Gruppe bestehend aus Bis(3,4-Dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure. Gemäß einer Ausführungsform der vorliegenden Erfindung ist die Verbindung der Formel (III) ausgewählt aus Bis(3,4-Dichlorphenyl)borinsäure und 4-Chlorphenylboronsäure.
   Wesentlich für eine hohe Ausbeute von Diphenylborinsäure (iii) ist der Einsatz von nur 0,7 Äq. Trialkylborat, bezogen auf das eingesetzte substituierte Chlorbenzol (IV). Bei einer Einsatzmenge von 1,1 Äq. Trialkylborat entsteht Phenylboronsäure, wie in der EP-A 0 888 261 beschrieben,
   Die Reaktionstemperatur bei dieser Verfahrensstufe liegt beispielsweise im Bereich von -20 bis 100°C, 20 bis 80°C oder 40 bis 60°C.
(iv) cyclische Boronsäureester der Formel (III), in welchen
   - m: 2 ist,
   - p: 1 ist,
   - Q¹ und Q²: jeweils unabhängig voneinander ausgewählt sind aus C₁₋₄-alkoxy Resten, die zusammen mit dem Boratom, an welches sie gebunden sind, einen 5- oder 6-gliedrigen Ring bilden, der mit C₁₋₄-Alkyl-Resten substituiert sein kann,
   R² und n wie oben definiert sind,
   sind gemäß Boronic Acids, Herausgeber Dennis G. Hall, Wiley-VCH 2005, S. 28ff und darin angegebenen Literaturstellen erhältlich.
   Als Beispiele für cyclische Boronsäureester, die gemäß der vorliegenden Erfindung verwendet werden können, seien Verbindungen gemäß den folgenden Formeln (iv-1) bis (iv-3) genannt: 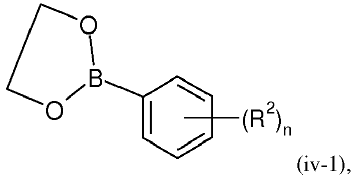 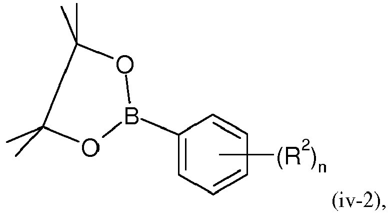 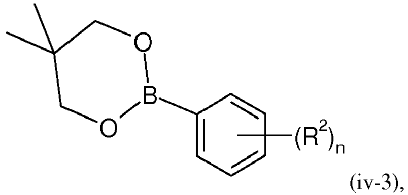 worin R² und n die oben angegebenen Bedeutungen haben.
(v) Boronate der Formel (III), in welchen
   - m: 3 ist,
   - p: 1 ist,
   - R² und n: wie oben definiert sind,
   - Q¹ bis Q³: jeweils unabhängig voneinander ausgewählt sind aus OH, F, Cl, Br, I, C₁₋₄-Alkyl-, C₆₋₁₀-Aryl-, C₁₋₄-Alkoxy- und C₆₋₁₀-Aryloxy-Resten,
   und worin die negative Ladung des Boronat Anions durch ein Kation kompensiert ist, wie durch die folgende Formel (iv-1) gezeigt.
   Das Kation (M⁺) ist beispielsweise aus der Gruppe bestehend aus Ammonium- (NH₄⁺), Alkali- oder Erdalkalimetallkationen, wie Na⁺, K⁺, Li⁺, Mg²⁺, Ca²⁺ ausgewählt.
   Die Boronate (v) sind gemäß Serwatowski et al., Tetrahedron Lett. 44, 7329 (2003), erhältlich.
(vi) Triarylborane der Formel (III), in welchen
   - m: 0 ist,
   - p: 3 ist,
   - R² und n: wie oben definiert sind.
   Die Triarylborane (vi) sind gemäß H.C. Brown et al., J. Organomet. Chem. 73, 1 (1988) und H.C. Brown et al., "Borane reagents", Verlag Harcourt Brace Jovanovich, (1988) erhältlich.
(vii) Tetraarylborate der Formel (III), in welchen
   - m: 0 ist,
   - p: 4 ist,

   - R² und n: wie oben definiert sind,
   und in welchen die negative Ladung des Boronat Anions durch ein Kation kompensiert ist welches beispielsweise aus der Gruppe bestehend aus Ammonium- (NH₄⁺), Alkali- oder Erdalkalimetall-Kationen, wie Na⁺, K⁺, Li⁺, Mg²⁺, Ca²⁺, ausgewählt ist.

Die Tetraarylborate (vii) sind gemäß J. Serwatowski et al., Tetrahedron Lett. 44, 7329 (2003), erhältlich.

### Suzuki Kupplung

Gemäß der vorliegenden Erfindung können chlorierte Biphenylanilide und Biphenylaniline der Formel (I) in hoher Selektivität und mit hohen Ausbeuten hergestellt werden.

Durch Einsatz von Phosphinliganden der allgemeinen Formel (V) 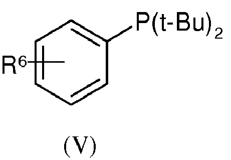 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂
oder ein Salz hiervon,
ist es im Vergleich zur Verwendung aliphatischer Phosphinliganden möglich, den Anteil an Triarylen zu vermindern. R¹ kann dabei in Formel (I) bzw. (II) eine geschützte Aminogruppe, NO₂, NH₂ und NHR³ sein, wobei R³ wie oben definiert ist.

Wie in WO 2009/135598 offenbart kann die Suzuki-Kupplung bei Schützung der Aminogruppe des Arylhalogenids der Formel (II) durch eine Schutzgruppe unter milderen Reaktionsbedingungen durchgeführt werden. Daher wird die Bildung von unerwünschten Nebenprodukten, wie Dehalogenierungsprodukten, Triarylen und polychlorierten Biphenylen (PCB), erheblich verringert.

Schutzgruppe bedeutet in diesem Zusammenhang jede Art von chemischer Gruppe, die zur Modifizierung der Aminogruppe des Arylhalogenids der Formel (II) während des Schritts der Suzuki-Kupplung verwendet und nach der Kupplung unter Rückbildung des ursprünglichen Amins von dem substituierten Biphenylanilid der Formel (I) abgespalten werden kann, beispielsweise durch Umsetzung mit wässriger Säure. Dieser Schritt wird als Entschützung bezeichnet.

Als Beispiele für Schutzgruppen, die im Allgemeinen für den Schutz von Amingruppen eingesetzt werden können, seien die folgenden Gruppen genannt:
Schiff-Basen (RR"C=N-R'), die durch Reaktion der Aminogruppe mit einem Aldehyd oder Keton erhalten werden. Die Abspaltung der Schiff-Base-Schutzgruppe kann beispielsweise durch Säurebehandlung, durch Hydrierung mit Pd/C/Wasserstoff gemäß J. Am. Chem. Soc. 1960, 82, 5688, oder mit Hydrazin in Ethanol gemäß J. Chem. Soc. C, 1969, 1758, erfolgen.

Bevorzugt verwendet man Ketone wie Aceton, Benzophenon oder Pinakolon oder Aldehyde wie Formaldehyd, Acetaldehyd oder Benzaldehyd.

Acetylamino- und Acetacetylaminogruppen werden durch Reaktion der Aminogruppe mit Essigsäure oder mit Acetessigsäureestern erhalten. Die Abspaltung der Gruppen kann durch Säurebehandlung erfolgen.

In einer Variante des beschriebenen Verfahrens ist die Aminogruppe des Arylhalogenids der Formel (II) durch eine Schiff-Base, durch eine Acetamino- oder durch eine Acetacetylaminogruppe geschützt.

In einer weiteren bevorzugten Variante des beschriebenen Verfahrens
ist X ausgewählt aus Wasserstoff, Fluor und Chlor;
ist R¹ ausgewählt aus -NH(CO)R³, -N=CR⁴R⁵ und NH₂;
steht R² für Chlor;
sind R³, R⁴, R⁵ unabhängig voneinander ausgewählt aus Wasserstoff, -CH₂-(C=O)-C₁₋₈-Alkyl, C₁₋₈-Alkyl, C₁₋₈-Alkenyl, C₁₋₈-Alkinyl und C₆₋₁₈-Aryl; oder
können R⁴, R⁵ zusammen mit dem Kohlenstoffatom, an das sie gebunden sind, einen fünf- oder sechsgliedrigen Ring mit einem, zwei oder drei aus N, O oder S ausgewählten Heteroatomen bilden; und
ist n ausgewählt aus 1, 2 und 3.

In einer anderen Variante des beschriebenen Verfahrens haben die nach dem Verfahren hergestellten substituierten Biphenyle die folgenden Substituenten, jeweils sowohl einzeln als auch in Kombination:
- X: ist ausgewählt aus Wasserstoff, Fluor und Chlor;
- R¹: ist ausgewählt aus -NH(CO)CH₃ und NH₂;
- R²: steht für Chlor;
- n: ist ausgewählt aus 1 und 2, vorzugsweise 2.

Die Durchführung der sich anschließenden homogen katalysierten Suzuku-Biaryl-Kreuzkupplung erfolgt gemäß Schema 1. 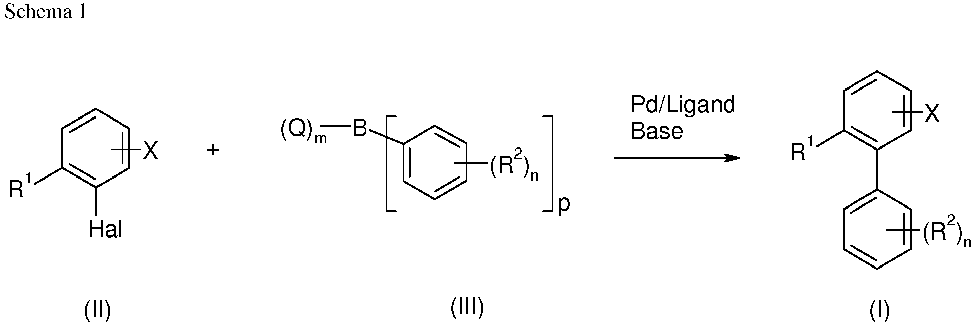 wobei als Ligand eine Verbindung der Formel (V) oder ein Salz hiervon eingesetzt wird. Die Substituenten sind wie oben beschrieben.

Der Phosphinligand der Formel (V) kann auch als Phosphonium-Salz wie z.B. als Tetrafluoroborat (Org. Lett. 2001, 3, 4295), Perchlorat oder Hydrogensulfat eingesetzt und hieraus *in situ* durch Base freigesetzt werden.

Die eingesetzte Base kann neben der Neutralisation der entstehenden Säure auch durch eine Aktivierung der Arylboronsäure zu anionischen Boronatspezies den Reaktionsverlauf positiv beeinflussen. Neben den oben genannten Basen kann eine solche Aktivierung auch durch Zusatz von Fluoridsalzen wie beispielsweise CaF, NaF, KF, LiF oder CsF erreicht werden.

Die eingesetzten Palladiumkatalysatoren werden in der Regel *in situ* aus mindestens einem Palladium(II)salz oder einer Palladium(0)-Verbindung und den entsprechenden Phosphin-Liganden erzeugt. Sie können jedoch auch als Palladium(0)-Verbindung direkt eingesetzt werden, ohne dass dadurch die anfängliche katalytische Aktivität gemindert wird.

Die unten stehenden Herstellungsbeispiele zeigen deutlich den überraschenden Vorteil des Aryl-Alkyl-Phosphinliganden der Formel (V), beispielsweise [(t-Bu)₂PhPH]BF₄, gegenüber einem Alkyl-Phosphinliganden, beispielsweise [(t-Bu)₃PH]BF₄. Der erfindungsgemäße Einsatz des Liganden der Formel (V) oder eines Salzes hiervon führt zu einer deutlich verringerten Bildung von Triarylen im Vergleich zum Einsatz von Alkyl-Phosphinliganden, beispielsweise [(t-Bu)₃PH]BF₄.

Beispiele der Verbindung (II) sind ausgewählt aus der Gruppe bestehend aus N-(2-Brom-4-fluorphenyl)acetamid, N-(2-Bromophenyl)acetamid, N-(2-bromphenyl)-3-oxobutanamid, N-(2-Bromo-4-fluorphenyl)-3-oxobutanamid, 2-Brom-N-(Propan-2-yliden)aniline, 2-Brom-4-Fluor-N-(Propan-2-yliden)anilin, N-(2-Brom-4-fluor)anilin, N-(2-bromo)anilin.

Eine Variante des oben beschriebenen Verfahrens sieht daher auch vor, dass die Verbindung (II) ausgewählt ist aus der Gruppe bestehend aus N-(2-Brom-4-fluorphenyl)acetamid, N-(2-Bromophenyl)acetamid, N-(2-bromphenyl)-3-oxobutanamid, N-(2-Bromo-4-fluorphenyl)-3-oxobutanamid, 2-Brom-N-(Propan-2-yliden)aniline, 2-Brom-4-Fluor-N-(Propan-2-yliden)anilin, N-(2-Brom-4-fluor)anilin, N-(2-bromo)anilin.

Die Verbindung (II) wird, bezogen auf die Organoborverbindung (III) (Boräquivalente), normalerweise äquimolar, vorzugsweise mit bis zu 20 prozentigem Überschuss, insbesondere mit bis zu 50 prozentigem Überschuss, ganz speziell mit bis zu 100 prozentigem Überschuss, verwendet.

Bevorzugte Verbindungen der Formel (III) umfassen sowohl Borinsäuren der Formel (III) (iii) als auch Boronsäuren der Formel (III) (i) oder (ii). Aus Gründen der Wirtschaftlichkeit ist der Einsatz von Borinsäuren der Formel (III) (iii) bevorzugt.

Beispiele für bevorzugte Verbindungen der Formel (III) sind ausgewählt aus der Gruppe bestehend aus Bis(3,4-Dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Bevorzugte Verbindungen der Formel (III) sind auch ausgewählt aus der Gruppe bestehend aus Bis(3,4-Dichlorphenyl)borinsäure, Bis(2,3-Dichlorphenyl)borinsäure, Bis(3-Dichlorphenyl)borinsäure, und Bis(4-Dichlorphenyl)borinsäure.

### Beispiele für Kombinationen von Verbindungen (II) und (III) sind:

Verbindung (II) ist 2-Brom-4-fluoracetanilid und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Verbindung (II) ist 2-Brom-4-fluoranilin und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Verbindung (II) ist 2-Brom-acetanilid und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Verbindung (II) ist 2-Bromanilin und Verbindung (III) ist ausgewählt aus der Gruppe bestehend aus Bis(3,4-dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

Gemäß einer weiteren Variante ist die Verbindung (II) 2-Brom-4-fluoracetanilid und die Verbindung (III) ist Bis(3,4-dichlorphenyl)borinsäure.

Gemäß einer weiteren Variante ist die Verbindung (II) 2-Brom-4-fluoranilin und die Verbindung (III) ist Bis(3,4-dichlorphenyl)borinsäure.

Gemäß einer weiteren Variante ist die Verbindung (II) 2-Brom-acetanilid und die Verbindung (III) ist 4-Chlorphenylboronsäure

Gemäß einer weiteren Variante ist die Verbindung (II) 2-Bromanilin und die Verbindung (III) ist 4-Chlorphenylboronsäure.

Als Basen können organische Basen, beispielsweise tertiäre Amine, eingesetzt werden. Bevorzugt verwendet man beispielsweise Triethylamin oder Dimethylcyclohexylamin. Als Basen verwendet man vorzugsweise Alkalimetallhydroxide, Erdalkalimetallhydroxide, Alkalimetallcarbonate, Erdalkalimetallcarbonate, Alkalimetallhydrogencarbonate, Alkalimetallacetate, Erdalkalimetallacetate, Alkalimetallalkoholate und Erdalkalimetallalkoholate, im Gemisch und insbesondere einzeln. Als Basen besonders bevorzugt sind Alkalimetallhydroxide, Erdalkalimetallhydroxide, Alkalimetallcarbonat, Erdalkalimetallcarbonat und Alkalimetallhydrogencarbonate. Als Basen insbesondere bevorzugt sind Alkalimetallhydroxide, z.B. Natriumhydroxid und Kaliumhydroxid, sowie Alkalimetallcarbonate und Alkalimetallhydrogencarbonate, z.B. Lithiumcarbonat, Natriumcarbonat und Kaliumcarbonat. Demnach ist gemäß einer Ausführungsform der vorliegenden Erfindung die Base ausgewählt aus Alkalimetall Hydroxiden, Alkalimetall Carbonaten und Alkalimetall Hydrogencarbonaten. Gemäß einer weiteren Ausführungsform der vorliegenden Erfindung ist die Base ausgewählt aus NaOH, KOH, Li₂CO₃, Na₂CO₃ und K₂CO₃. Die Base wird bei dem erfindungsgemäßen Verfahren vorzugsweise mit einem Anteil von 100 bis 500 mol-%, weiter bevorzugt 150 bis 400 mol-%, bezogen auf die Menge von Organoborverbindung (III), eingesetzt.

Geeignete Palladiumkatalysatoren sind Palladium-Ligand-Komplexe mit Palladium in der Oxidationsstufe Null, Palladiumsalze in Gegenwart von Komplexliganden oder gegebenenfalls auf Träger aufgezogenes metallisches Palladium, in Anwesenheit von Phosphinliganden der allgemeinen Formel (V) 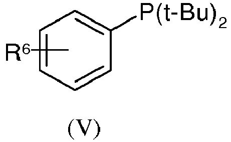 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂,
oder ein Salz hiervon.

Gemäß einer weiteren Ausführungsform der vorliegenden Erfindung ist
- R⁶: ausgewählt aus Wasserstoff, Methyl, Difluormethyl und Trifluormethyl, und
- R⁷: ist (CH₃)₂.

Gemäß einer Ausführungsform der vorliegenden Erfindung sind die Phosphinliganden der allgemeinen Formel (V) ausgewählt aus Di(tert.-butyl)phenylphosphin, Di-tert-butyl-p-[4-(trifluoromethyl)phenyl]phosphin, 4-(Di-tert-butylphosphino)-p-N,N-dimethylanilin und Di-tert-butyl-p-(4-methylphenyl)phosphin.

Gemäß einer weiteren Ausführungsform der vorliegenden Erfindung ist der Phosphinligand der allgemeinen Formel (V) Di(tert.-butyl)phenylphosphin.

Gegenstand der vorliegenden Erfindung ist auch eine Verbindung der Formel (V) 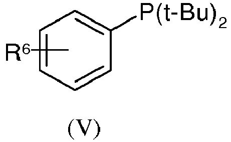 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂.

Ein weiterer Gegenstand der vorliegenden Erfindung ist eine Verbindung der Formel (V) 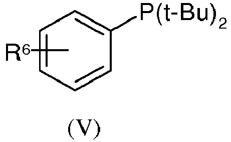 wobei
- R⁶: ausgewählt ist aus Wasserstoff, Methyl, 1- bis 3-fach halogeniertem Methyl und NR⁷ und
- R⁷: (CH₃)₂ ist.

Die in Formel (V) dargestellten Phosphine können auch in Form ihrer Salze, wie z. B. als Tetrafluoroborat, Perchlorat oder Hydrogensulfat eingesetzt werden und durch Zusatz einer Base *in situ* freigesetzt werden.

Ein weiterer Gegenstand der vorliegenden Erfindung ist eine Verbindung der Formel (V-i) 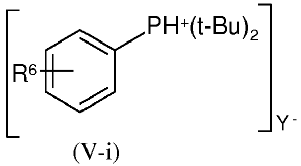 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂ und
- Y: ausgewählt ist aus der Gruppe bestehend aus BF₄⁻, Perchlorat und Hydrogensulfat.

Gemäß einer Ausführungsform der vorliegenden Erfindung ist der Palladium Katalysator ausgewählt aus der Gruppe bestehend aus:
a) einem Komplex bestehend aus Palladium in der Oxidationsstufe 0 und einem Phosphinliganden der Formel (V) oder einem Salz hiervon,
b) einem Palladiumsalz in Gegenwart eines Phosphinliganden der Formel (V) oder einem Salz hiervon und
c) metallischem, gegebenenfalls auf einen Träger aufgebrachtem Palladium, in Gegenwart eines Phosphinliganden der Formel (V) oder einem Salz hiervon.

In einer Ausführungsform der vorliegenden Erfindung ist der Palladium Katalysator a) ein Komplex aus Palladium im Oxidationszustand 0 und einem Phosphinliganden der Formel (V) oder ein Salz hiervon ist.

In einee weiteren Ausführungsform der vorliegenden Erfindung ist das Salz des Palladium Katalysators b) ausgewählt aus der Gruppe bestehend aus Palladiumchlorid, Palladiumacetat, Palladiumacetylacetonat und Bisacetonitril-Palladiumchlorid.

In einer weiteren Ausführungsform der vorliegenden Erfindung besteht der Palladium Katalysator c) aus metallischem Palladium auf aktiviertem Kohlenstoff in Anwesenheit eines Phosphinliganden der allgemeinen Formel (V) oder eines Salzes hiervon.

Die Reaktivität der Komplexliganden kann durch Zusatz eines quartären Ammoniumsalzes wie Tetra-n-butylammoniumbromid (TBAB) gesteigert werden (siehe beispielsweise D. Zim et al., Tetrahedron Lett. 2000, 41, 8199). Bei Bedarf kann die Wasserlöslichkeit der Palladiumkomplexe durch verschiedene Substituenten verbessert werden, wie Sulfonsäure- oder Sulfonsäuresalzgruppen, Carbonsäure- oder Carbonsäuresalzgruppen, Phosphonsäure-, Phosphonium- oder Phosphonsäuresalzgruppen, Peralkylammonium-, Hydroxy- und Polyethergruppen.

Aus den Palladium-Ligand-Komplexen mit Palladium in der Oxidationsstufe Null verwendet man vorzugsweise Tetrakis(triphenylphosphin)palladium und daneben Tetrakis[tri(o-tolyl)phosphin]palladium. In den Palladiumsalzen, die in Gegenwart von Komplexliganden verwendet werden, liegt das Palladium normalerweise in der zweifach positiven Oxidationsstufe vor. Gemäß einer bevorzugten Ausführungsform der vorliegenden Erfindung ist der Palladium Katalysator b) ausgewählt aus der Gruppe bestehend aus palladium chloride, palladium acetate, Palladiumacetylacetonat or bisacetonitrilepalladium chloride. Particular preference is given to using Palladiumacetylacetonat.

Das molare Verhältnis von Palladium zum Phosphin-Liganden der Formel (V) oder eines seiner Salze sollte zwischen 4:1 und 1:100 liegen, und liegt vorzugsweise zwischen 1 : 1 und 1 : 5, besonders bevorzugt zwischen 1 : 1 und 1 : 2.

Besonders bevorzugt ist bei der Verwendung von gegebenenfalls auf Träger aufgezogenem metallischem Palladium die Mitverwendung der vorstehend genannten Phosphinliganden der Formel (V) oder (V-i). Der Palladiumkatalysator wird bei dem erfindungsgemäßen Verfahren mit einem niedrigen Anteil von 0,001 bis 1,0 mol-%, vorzugsweise 0,005 bis 0,5 mol-% oder 0,01 bis 0,5 mol-% und insbesondere 0,005 bis 0,05 mol-%, bezogen auf die Menge von Verbindung (II), eingesetzt.

Das erfindungsgemäße Verfahren kann in einem Zweiphasensystem aus wässriger Phase und fester Phase, d.h. dem Katalysator, durchgeführt werden. Die wässrige Phase kann dabei neben Wasser auch ein wasserlösliches organisches Lösungsmittel enthalten.

Für das erfindungsgemäße Verfahren geeignete organische Lösungsmittel sind Ether, wie Dimethoxyethan, Diethylenglykoldimethylether, Tetrahydrofuran, Dioxan und tert.-Butylmethylether, Kohlenwasserstoffe wie n-Hexan, n-Heptan, Cyclohexan, Benzol, Toluol und Xylol, Alkohole wie Methanol, Ethanol, 1-Propanol, 2-Propanol, Ethylenglykol, 1-Butanol, 2-Butanol und tert.-Butanol, Ketone wie Aceton, Ethylmethylketon und Isobutylmethylketon, Amide wie Dimethylformamid, Dimethylacetamid und N-Methylpyrrolidon, jeweils einzeln oder im Gemisch.

Bevorzugte Lösungsmittel sind Ether, wie Dimethoxyethan, Tetrahydrofuran und Dioxan, Kohlenwasserstoffe wie Cyclohexan, Toluol und Xylol, Alkohole wie Ethanol, 1- Propanol, 2-Propanol, 1-Butanol und tert.-Butanol, jeweils einzeln oder im Gemisch. In einer besonders bevorzugten Variante des erfindungsgemäßen Verfahrens verwendet man ein oder mehrere wasserunlösliche und ein oder mehrere wasserlösliche Lösungsmittel, beispielsweise Gemische aus Wasser und Dioxan oder Wasser und Tetrahydrofuran oder Wasser, Dioxan und Ethanol oder Wasser, Tetrahydrofuran und Methanol oder Wasser, Toluol und Tetrahydrofuran, vorzugsweise Wasser und Tetrahydrofuran oder Wasser, Tetrahydrofuran und Methanol.

In einer Variante des oben beschriebenen Verfahrens wird die Reaktion in einer Mischung aus Wasser und einem organischen Lösungsmittel durchgeführt. In einer weiteren Variante des Verfahrens wird die Reaktion in einer Mischung aus Wasser und 1-Butanol durchgeführt.

Die Gesamtmenge an Lösungsmittel liegt normalerweise bei 3000 bis 500 g und vorzugsweise 2000 bis 700 g pro Mol der Verbindung (II).

Zweckmäßigerweise werden zur Durchführung des Verfahrens die Verbindung (II), die Organoborverbindung (III), die Base und die katalytisch wirksame Menge des Palladiumkatalysators in ein Gemisch aus Wasser und einem oder mehreren inerten organischen Lösungsmitteln gegeben und bei einer Temperatur von 20°C bis 100°C, vorzugsweise 50°C bis 90°C, weiter bevorzugt 60°C bis 80°C, über einen Zeitraum von 1 bis 50 Stunden, vorzugsweise 2 bis 24 Stunden, gerührt.

Je nach verwendetem Lösungsmittel und verwendeter Temperatur stellt sich ein Druck von 1 bar bis 6 bar, vorzugsweise 1 bar bis 4 bar, ein. Bevorzugt wird die Umsetzung in Wasser und Tetrahydrofuran durchgeführt. Die Umsetzung kann in üblichen Apparaturen, die für derartige Verfahren geeignet sind, durchgeführt werden. Nach beendeter Umsetzung wird als Feststoff anfallender Palladiumkatalysator beispielsweise durch Filtration abgetrennt und das Rohprodukt vom Lösungsmittel bzw. den Lösungsmitteln befreit. Bei nicht völlig wasserlöslichen Produkten werden wasserlösliche Palladiumkatalysatoren oder Komplexliganden bei der Trennung der Wasserphase vom Rohprodukt vollständig abgetrennt. Anschließend kann nach dem Fachmann bekannten und dem jeweiligen Produkt angemessenen Methoden eine weitere Aufreinigung erfolgen, beispielsweise durch Umkristallisation, Destillation, Sublimation, Zonenschmelzen, Schmelzekristallisation oder Chromatographie.

Mit dem beschriebenen Verfahren können beispielsweise die folgenden Verbindungen hergestellt werden:
N-(3',4'-Dichlor-5-fluorbiphenyl-2-yl)acetamid, 3',4'-Dichlor-5-fluorbiphenyl-2-amin, N-(4'-Chlorbiphenyl-2-yl)acetamid, 4'-Chlorbiphenyl-2-amin.

Weitere Beispiele sind: 3',4'-Dichlor-5-fluor-N-(propan-2-yliden)biphenyl-2-amin, 3',4'-Dichlor-N-(propan-2-yliden)biphenyl-2-amin, 4'-Chlor-N-(propan-2-yliden)biphenyl-2-amin, N-(4'-Chlor-5-fluorbiphenyl-2-yl)acetamid, N-(3',4'-Dichlor-biphenyl-2-yl)acetamid.

Das beschriebene Verfahren liefert die Verbindungen der Formel (I) in sehr hohen quantitativen Ausbeuten bei sehr guter Reinheit. Die mit dem erfindungsgemäßen Verfahren erhältlichen substituierten Biphenyle eignen sich als Vorprodukte für fungizide Pflanzenschutz-Wirkstoffe (siehe WO 03/070705). Bei Verwendung einer Amin-Schutzgruppe wird diese in den meisten Fällen wird die Amin-Schutzgruppe vor der weiteren Umwandlung der Amine abgespalten.

### Allgemeine Verfahrensvarianten

Beschrieben wird ferner ein Verfahren zur Herstellung halogenierter Biphenylanilide der Formel (I) 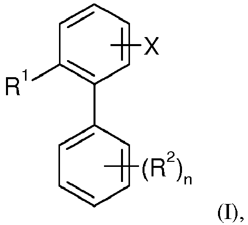 worin
- X: ausgewählt ist aus Wasserstoff, Fluor und Chlor;
- R¹: ausgewählt ist aus -NH(CO)R³, -N=CR⁴R⁵, NO₂, NH₂ und NHR³;
- R²: Chlor ist;
- R³, R⁴, R⁵: unabhängig voneinander ausgewählt sind aus Wasserstoff, -CH₂-(C=O)CH₃, C₁-C₈-Alkyl, C₁-C₈-Alkenyl, C₁-C₈-Alkynyl und C₆-C₁₈-Aryl; oder
- R⁴, R⁵: zusammen mit dem Kohlenstoffatom, an das sie gebunden sind einen 5- oder 6-gliedrigen Ring bilden können, der 1, 2 oder 3 Heteroatome ausgewählt aus N, O, und S enthält;
- n: ausgewählt ist aus 1, 2 und 3,
durch Umsetzung einer Verbindung der Formel (II) 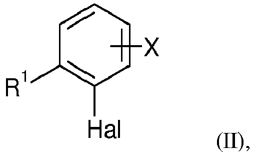 worin
Hal ausgewählt ist aus Brom und Jod; und R¹ und X wie oben definiert sind,
in Gegenwart einer Base und eines Palladium Katalysators ausgewählt aus der Gruppe bestehend aus:
a) einem Komplex bestehend aus Palladium in der Oxidationsstufe 0 und einem Phosphinliganden der Formel (V) oder einem Salz hiervon,
b) einem Palladiumsalz in Gegenwart eines Phosphinliganden der Formel (V) oder einem Salz hiervon und
c) metallischem, gegebenenfalls auf einen Träger aufgebrachtem Palladium, in Gegenwart eines Phosphinliganden der Formel (V) oder einem Salz hiervon,
wobei der Phosphinligand der Formel (V) wie folgt definiert ist 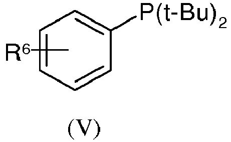 wobei
- R⁶: ausgewählt ist aus Wasserstoff, C₁-C₄ Alkyl, C₁-C₄ Haloalkyl, Phenyl und NR⁷ und
- R⁷: ausgewählt ist aus (C₁-C₄-Alkyl)₂,
oder ein Salz hiervon
in einem Lösungsmittel, mit einer Organoborverbindung der Formel (III) 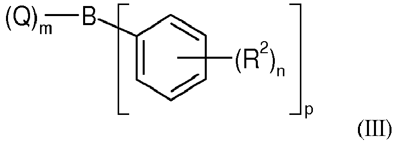 ausgewählt aus der Gruppe bestehend aus:
(i) Boronsäuren der Formel (III) in welchen
   - m: 2 ist,
   - p: 1 ist,
   - Q¹ und Q²: Hydroxylgruppen sind,
   - R² und n: wie oben definiert sind,
   oder die aus den Boronsäuren der Formel (III) gebildeten Anhydride, Dimere oder Trimere;
(ii) Boronsäurederivate der Formel (III), in welchen
   - m: 2 ist,
   - p: 1 ist,
   Q¹ und Q² jeweils unabhängig voneinander ausgewählt sind aus F, Cl, Br, I, C₁₋₄-Alkyl-, C₆₋₁₀-Aryl-, C₁₋₄-Alkoxy- und C₆₋₁₀-Aryloxy sind,
   R² und n wie oben definiert sind;
(iii) Borinsäuren der Formel (III), in welchen
   - m: 1 ist,
   - p: 2 ist,
   - Q: ausgewählt ist aus OH, F, Cl, Br, I, C₁₋₄-Alkyl-, C₆₋₁₀-Aryl-, C₁₋₄-Alkoxy- und C₆₋₁₀-Aryloxy-Resten,
   - R² und n: wie oben definiert sind;
(iv) cyclische Boronsäureester der Formel (III), in welchen
   - m: 2 ist,
   - p: 1 ist,
   - Q¹ und Q²: jeweils unabhängig voneinander ausgewählt sind aus C₁₋₄-Alkoxy Resten, die zusammen mit dem Boratom, an welches sie gebunden sind, einen 5- oder 6-gliedrigen Ring bilden, der mit C₁₋₄-Alkyl-Resten substituiert sein kann,
   - R² und n: wie oben definiert sind;
(v) Boronate der Formel (III), in welchen
   - m: 3 ist,
   - p: 1 ist,
   - R² und n: wie oben definiert sind,
   - Q¹ bis Q³: jeweils unabhängig voneinander ausgewählt sind aus OH, F, Cl, Br, I, C₁₋₄-Alkyl-, C₆₋₁₀-Aryl-, C₁₋₄-Alkoxy- und C₆₋₁₀-Aryloxy-Resten,
   und worin die negative Ladung des Boronat Anions durch ein Kation kompensiert ist;
(vi) Triarylborane der Formel (III), in welchen
   - m: 0 ist,
   - p: 3 ist,
   - R² und n: wie oben definiert sind;
(vii) Tetraarylborate der Formel (III), in welchen
   - m: 0 ist,
   - p: 4 ist,
   - R² und n: wie oben definiert sind,
   und in welchen die negative Ladung des Boronat Anions durch ein Kation kompensiert ist.

In einer Variante des Verfahrens ist Verbindung (II) ausgewählt aus der Gruppe bestehend aus N-(2-Brom-4-fluorphenyl)acetamid, N-(2-Bromophenyl)acetamid, N-(2-bromphenyl)-3-oxobutanamid, N-(2-Bromo-4-fluorphenyl)-3-oxobutanamid, 2-Brom-N-(Propan-2-yliden)aniline, 2-Brom-4-Fluor-N-(Propan-2-yliden)anilin, N-(2-Brom-4-fluor)anilin, N-(2-bromo)anilin.

In einer weiteren Variante des Verfahrens ist die Verbindung der Formel (III) ausgewählt aus der Gruppe bestehend aus Bis(3,4-Dichlorphenyl)borinsäure, Bis(2,3-Dichlorphenyl)borinsäure, Bis(3-Dichlorphenyl)borinsäure, Bis(4-Dichlorphenyl)borinsäure, 4-Chlorphenylboronsäure, 3-Chlorphenylboronsäure, 2-Chlorphenylboronsäure, 3,4-Dichlorphenylboronsäure und 2,3-Dichlorphenylboronsäure.

In einer weiteren Variante des Verfahrens ist die Base ausgewählt aus Alkalimetall Hydroxiden, Alkalimetall Carbonaten und Alkalimetall Hydrogencarbonaten.

In einer weiteren Variante des Verfahrens ist der Palladium Katalysator a) gemäß der oben gemachten Beschreibung ein Komplex aus Palladium im Oxidationszustand 0 und einem Phosphinliganden der Formel (V) oder einem Salz hiervon.

In einer weiteren Variante des Verfahrens wird ein Palladium Katalysator b) gemäß der oben gemacht Beschreibung verwendet.

In einer weiteren Variante des Verfahrens wird ein Palladium Katalysator c) gemäß der oben gemachten Beschreibung verwendet und besteht dieser Palladium Katalysator c) aus metallischem Palladium auf aktiviertem Kohlenstoff in Anwesenheit eines Phosphinliganden der allgemeinen Formel (V) oder einem Salz hiervon.

In einer weiteren Variante des Verfahrens ist das Salz des Palladium Katalysators b) ausgewählt aus der Gruppe bestehend aus Palladiumchlorid, Palladiumacetate, Palladiumacetylacetonat und Bisacetonitril-Palladiumchloride.

In einer weiteren Variante des Verfahrens ist der Phosphinligand der allgemeinen Formel (V) ausgewählt aus Di(tert.-butyl)phenylphosphin, Di-tert-butyl p-[4-(trifluoromethyl)phenyl]phosphin, 4-(Di-tert-butylphosphino)-p-N,N-dimethylanilin und Di-tert-butyl-p-(4-methylphenyl)phosphin.

In einer weiteren Variante des Verfahrens wird ein Palladium Katalysator b) verwendet, wobei das molare Verhältnis des Palladium Salzes zu dem Phosphinliganden der allgemeinen Formel (V) oder einem Salz hiervon 1 : 1 bis 1 : 5 beträgt..

In einer weiteren Variante des Verfahrens werden 0.001 bis 1.0 mol% des Palladium Katalysators bezogen auf die Menge der Verbindung der Formel (II) verwendet.

In einer weiteren Variante des Verfahrens wird die Reaktion bei einer Temperatur von 20 bis 100°C durchgeführt.

In einer weiteren Variante des Verfahrens wird die Reaktion in einer Mischung aus Wasser und einem organischen Lösungsmittel durchgeführt.

### Herstellungsbeispiele

### Allgemeine Arbeitsvorschrift zur Biarylsynthese

Unter Argon wird eine Mischung aus Anilin bzw. Acetanilid (1,0 Äquivalent), chlorierte Diphenylborinsäure 0,5 Äquivalente) od. chlorierte Phenylboronsäure (1,0 Äquivalent), Base, [(t-Bu)3PH]BF4 bzw. [(t-Bu)2PhPH]BF4 (0,12 mol%), Pd(acac)2 (0,12 mol%) in 8 ml Wasser und 2 ml 1-Butanol auf 60°C erwärmt. Die Reaktionsmischung wird ca. 20 Stunden bei 60°C gerührt, auf Raumtemperatur abgekühlt und mit 1 N Salzsäure angesäuert. Nach zweimaliger Extraktion der Reaktionsmischung mit Ethylacetat werden die vereinigten organischen Phasen über Magnesiumsulfat getrocknet. Das Lösungsmittel wird im Vakuum abdestilliert.

Entsprechend der allgemeinen Arbeitsvorschrift wurden folgende Biaryle hergestellt:
1. *N*-(3',4'-dichloro-5-fluorobiphenyl-2-yl)acetamid (Biaryl 1)
2. *3',4*'-Dichlor-5-fluorbiphenyl-2-amin (Biaryl 2)
3. *N*-(4'-Chlorbiphenyl-2-yl)acetamid (Biaryl 3)
4. *4*'-Chlorbiphenyl-2-amin (Biaryl 4)

Aus Tabelle 1 gehen Ansatzgröße und Reagenzien hervor.

**Tabelle 1**

| **Biaryl** | **Anilin/Anilid** | **Ansatzgröße [mmol]** | **Borverbindung** | **Base** | **Äquivalente Base** |
|---|---|---|---|---|---|
| **1** | A-1 | 4,3 | B-1 | K₂CO₃ | 1,74 |
| **2** | A-2 | 5,7 | B-1 | K₂CO₃ | 1,74 |
| **3** | A-3 | 4,6 | B-2 | Phosphatpuffer pH 7-10 | 2,18 20ml / 0,5M |
| **4** | A-4 | 5,7 | B-2 | K₂CO₃ | 1,74 |

### Anilin/Anilid

A-1: 2-Brom-4-fluoracetanilid
A-2: 2-Brom-4-fluoranilin
A-3: 2-Brom-acetanilid
A-4: 2-Bromanilin

### Borverbindung

### B-1: Bis(3,4-dichlorphenyl)borinsäure

### B-2: (4-Chlorphenyl)boronsäure

Tabelle 2 zeigt den Anteil (Fl.%, GC-MS) gebildeter Triaryle in der Reaktionsmischung bei Herstellung der Biaryle 1 bis 4 in Abhängigkeit des verwendeten Liganden.

**Tabelle 2**

| **Biaryl** | **1** | | **2** | | **3** | | **4** | |
|---|---|---|---|---|---|---|---|---|
| **Ligand** | A | B | A | B | A | B | A | B |
| **Triaryl [Fl.%, GC-MS]** | 5,6 | 3,3 | 2,1 | 0,9 | 0,3 | 0 | 3,5 | 0,5 |

| | | | | | | | | |
|---|---|---|---|---|---|---|---|---|
| Ligand A: [(t-Bu)₃PH]BF₄ Ligand B: [(t-Bu)₂PhPH]BF₄ | | | | | | | | |

### Beispiel

### Herstellung von N-(3',4'-dichloro-5-fluorobiphenyl-2-yl)acetamid

Unter Argon wurde eine Mischung aus *N*-(2-bromo-4-fluorophenyl)acetamid (1,00 g, 4,27 mmol), Bis(3,4-dichlorphenyl)borinsäure (0,685 g, 2,14 mmol), Kaliumcarbonat (1,03 g, 7,44 mmol), [(t-Bu)₂PhPH]BF₄ (1,6 mg, 5,2 µmol), Pd(acac)₂ (1,6 mg, 5,3 µmol) in 8 ml Wasser und 2 ml 1-Butanol auf 60°C erwärmt. Die Reaktionsmischung wurde ca. 13 Stunden bei 60°C gerührt, auf Raumtemperatur abgekühlt und mit 1 N Salzsäure angesäuert. Nach zweimaliger Extraktion der Reaktionsmischung mit Ethylacetat wurden die vereinigten organischen Phasen über Magnesiumsulfat getrocknet. Das Lösungsmittel wurde im Vakuum abdestilliert. Es wurden 1,21 g Rohprodukt erhalten (90,8 Fl.% HPLC, 86 % Ausbeute).

## Patentansprüche

1. Verbindung der Formel (V) 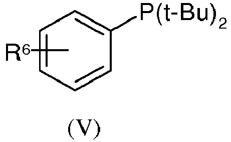 wobei
R⁶ ausgewählt ist aus Wasserstoff, C₁-C₄ Alkyl, C₁-C₄ Haloalkyl, Phenyl und NR⁷ und
R⁷ ausgewählt ist aus (C₁-C₄-Alkyl)₂.

2. Salz der Formel (V-i) 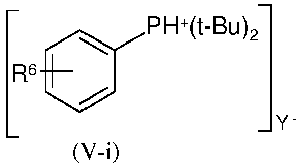 wobei
R⁶ ausgewählt ist aus Wasserstoff, C₁-C₄ Alkyl, C₁-C₄ Haloalkyl, Phenyl und NR⁷ und
R⁷ ausgewählt ist aus (C₁-C₄-Alkyl)₂ und
Y ausgewählt ist aus der Gruppe bestehend aus BF₄⁻, Perchlorat und Hydrogensulfat.

3. Salz nach Anspruch 2, wobei die Verbindung der Formel (V-i) eine Verbindung der Formel [(t-Bu)₂PhPH]BF₄ ist.
